# EUROPEAN PATENT APPLICATION

(11) **EP 4 332 583 A1**
(43) Date of publication of application: **06.03.2024**
(21) Application number: 22794206.7
(22) Date of filing: 19.01.2022
(51) Int. Cl.: G01N 35/04, G01N 1/38, G01N 33/53

(54) **REAGENT MIXING AND LOADING DEVICE AND IMMUNOLOGICAL DETECTION APPARATUS**

(30) Priority: 28.04.2021 CN 202110467052
(71) Applicant: Shenzhen Yhlo Biotech Co., Ltd, 518116 Shenzhen (CN)
(72) Inventor: LAI, Yunfei, Shenzhen, Guangdong 518116 (CN); HUANG, Xiutao, Shenzhen, Guangdong 518116 (CN); ZHANG, Fuxing, Shenzhen, Guangdong 518116 (CN); XIAO, Yujin, Shenzhen, Guangdong 518116 (CN)
(74) Representative: Mewburn Ellis LLP
(86) International application number: PCT/CN2022/072672
(87) International publication number: WO 2022/227724

(57) **Abstract**

A reagent mixing and loading device (10) and an immunological detection apparatus. The reagent mixing and loading device (10) comprises a transmission mechanism (100), mixing mechanisms (200), a loading mechanism (300) and a driving mechanism (400), wherein the transmission mechanism (100) comprises a first rotating member (110) and a second rotating member (120), the first rotating member (110) being rotatably connected to a plurality of mixing mechanisms (200) arranged at intervals, and the first rotating member (110) being capable of synchronously rotating with the second rotating member (120); the driving mechanism (400) is used to drive the second rotating member (120) to rotate; each mixing mechanism (200) comprises a mixing column (210), a mixing transmission member (220) and a mixing limiting member (230), the mixing transmission member (220) being connected to the mixing column (210) in a sleeved manner and fitting with the second rotating member (120) in an abutting manner; and the loading mechanism (300) comprises a loading box (310) and a loading fitting member (320), the loading box (310) being provided with a reagent hole for the loading box (310) to be placed therein, and the loading fitting member (320) being rotatably arranged in the reagent hole and used for carrying the loading box (310). The reagent mixing and loading device (10) can implement the normal mixing of reagents without the occurrence of problems such as jamming and collision.

## Description

### TECHNICAL FIELD

The present disclosure relates to the field of biological detection and diagnosis, in particular to a reagent mixing and loading device and an immunological detection apparatus.

### BACKGROUND

With the development of modern science, immunodiagnostic technology has gradually become a key means of modern clinical testing and scientific research. Immunodiagnostic technology is an important diagnostic tool for tumor, diabetes, abnormal gonadal secretion and other diseases. Compared with conventional detection methods, which have shortcomings such as complicated manual operation, long reaction time and environmental pollution, immunodiagnostic technology, such as chemiluminescent immunoassay technology has the advantages like short reaction time, simple operation and high diagnostic efficiency, and it has been widely applied in modern medicine and scientific research fields.

Currently, the chemiluminescent immunoassay system mainly includes: a cuvette storage and transfer system, a sample loading system, a reagent loading system, a sample feeding system, an incubation system, a centrifugal cleaning system and a luminescent reading system. The reagent loading system needs to manually load and unload the reagent kit in the shutdown state, which stops the running test and affects the test time and results. Meanwhile, during the test, some reagents are used up, and different reagents are required for different test items. In these cases, reagent loading or reagent replacement is required. Currently, manual loading or replacement of reagents requires the shutdown operation, and reloading is prone to problems such as stuck and collision, which affects the ongoing test.

### SUMMARY

Based on the above, it is necessary to provide a reagent mixing and loading device and an immunological detection apparatus, which can not only realize the normal mixing of reagents, but also realize the smooth introduction of reagent kit without stuck and collision to speed up the test progress.

The device for mixing and loading reagents includes a transmission mechanism, mixing mechanisms, a loading mechanism and a driving mechanism. The transmission mechanism includes a first rotating member and a second rotating member. The first rotating member is rotatably connected to a plurality of spaced apart mixing mechanisms. The first rotating member is sleeved on the second rotating member and is capable of rotating synchronously with the second rotating member. The driving mechanism is connected to the second rotating member to drive the second rotating member to rotate. The mixing mechanism includes a mixing column, a mixing transmission member, and a mixing limiting member. The mixing transmission member is sleeved on and connected to the mixing column, and the mixing transmission member abuts against and is engaged with the second rotating member, so as to rotate in an opposite direction relative to the second rotating member. The loading mechanism includes a loading box and a loading fitting member. The loading box is provided with a reagent hole to place the loading box. The loading fitting member is rotatably disposed in the reagent hole to carry the loading box, and the mixing limiting member is capable of being engaged with the loading fitting member and pushing the loading fitting member to rotate.

In one of the embodiments, the mixing limiting member includes at least two spaced apart elastic limiting arms. The elastic limiting arm is connected to the mixing column. The loading fitting member is provided with at least two spaced apart loading limiting arms. The loading limiting arm is located at least partially outside the reagent hole. When the loading limiting arm is misaligned with the elastic limiting arm, the loading limiting arm abuts against the elastic limiting arm.

In one of the embodiments, the loading fitting member further includes a bearing plate. The bearing plate is rotatably provided in the reagent hole, and the loading limiting arm is connected to the bearing plate and protrudes from the reagent hole.

In one of the embodiments, a plurality of loading limiting arms are provided. The plurality of loading limiting arms are equally spaced apart on a first circumference.

In one of the embodiments, a plurality of elastic limiting arms are provided. The plurality of elastic limiting arms are equally spaced apart on a second circumference.

In one of the embodiments, a maximum diameter of the first circumference is less than a maximum diameter of the second circumference and greater than a minimum diameter of the second circumference. An inner wall of an end of the elastic limiting arm facing the loading limiting arm is provided with a guiding yielding surface, and the guiding yielding surface is in a shape of an outward slope.

In one of the embodiments, the second rotating member is a gear, the mixing transmission member is a mixing gear, and the second rotating member is engaged with the mixing transmission member.

In one of the embodiments, the transmission mechanism further includes a first transmission wheel, a transmission belt, and a second transmission wheel, the first transmission wheel is connected to the second transmission wheel through the transmission belt. The second transmission wheel is connected to a driving shaft of the driving mechanism. The second transmission wheel is connected to the second rotating member through a transmission shaft.

In one of the embodiments, a plurality of loading mechanisms are provided. The device for mixing and loading reagents further includes a loading transfer mechanism connected to the loading box to drive the loading box to move.

An embodiment of the present disclosure further provides an immunological detection apparatus including the reagent mixing and loading device.

The above-mentioned reagent mixing and loading device can not only realize the normal mixing of reagents, but also realize the smooth introduction of reagent kit without stuck and collision to speed up the test progress.

In summary, the reagent mixing and loading device has the following beneficial effects:
(1) The mixing mechanism is simple in structure, and the mixing transmission member of the mixing mechanism has elasticity. In actual production, it can be produced by opening a mold taking the advantage of the elasticity of plastic materials, and a large-scale production can be performed by opening a plastic mold, which is low in price and cost.
(2) Without considering the transmission error and strictly limiting the transmission ratio of the large and small gears (i.e., the second rotating member and the mixing transmission member), and regardless of the relative state of the elastic limiting arm and the loading limiting arm of the loading mechanism, the loading and introduction of the loading box can be realized without stuck and collision.
(3) In the above device for mixing and loading reagents, the maximum diameter of the first circumference is less than the maximum diameter of the second circumference and greater than the minimum diameter of the second circumference. In this way, it can be ensured that when the loading limiting arm is loading, an entering position of the mixing transmission member is within the end surface of the elastic limiting arm. Since the inner wall of the end of the elastic limiting arm facing the loading limiting arm has the guiding yielding surface, the loading limiting arm can gradually move downward along the slope of the guiding yielding surface to push away the elastic limiting arm. With the rotation of the mixing column, the loading limiting arm and the elastic limiting arm can gradually be in a mutually misaligned and clamped state. At this time, the squeezing force exerted by the loading limiting arm on the elastic limiting arm disappears, and the elastic limiting arm is reset. With the rotation of the mixing column, the elastic limiting arm pushes the loading limiting arm to rotate, so as to achieve mixing.
(4) During loading, the forces exerted on the elastic limiting arm is gradually reduced by multiple small distance movements of the loading limiting arm, and the deformation amount is also gradually reduced, so that the elastic limiting arm is prevented from being directly subjected to a relatively large impact force, the reagent loading is smoother, and the service life of the elastic limiting arm is also prolonged.
(5) The plurality of loading mechanisms can improve the efficiency of reagent loading and realize loading of multiple reagent kits. For example, during the test, some reagents are used up, and different reagents are required for different test items. In these cases, reagent loading or reagent replacement is required. At this time, no shutdown is required. Different reagent kit can be loaded through the gripping mechanism of reagent kit. The loading is not prone to issues such as stuck and collision, which does not affect ongoing testing.

### BRIEF DESCRIPTION OF THE DRAWINGS

FIG. 1 is a schematic view of a reagent mixing and loading device according to an embodiment of the present disclosure.
FIG. 2 is a schematic view of a loading fitting member directly aligned with a mixing limiting member according to an embodiment of the present disclosure.
FIG. 3 is a schematic view of the loading fitting member completely misaligned with the mixing limiting member according to an embodiment of the present disclosure.
FIG. 4 is a partial schematic view of the mixing limiting member according to an embodiment of the present disclosure.

### Description of reference signs:

10, reagent mixing and loading device; 100, transmission mechanism; 110, first rotating member; 120, second rotating member; 200, mixing mechanism; 210, mixing column; 220, mixing transmission member; 230, mixing limiting member; 231, elastic limiting arm; 2311, guiding yielding surface; 300, loading mechanism; 310, loading box; 320, loading fitting member; 321, loading limiting arm; 400, driving mechanism.

### DETAILED DESCRIPTION

In order to make the objectives, features, and advantages of the present disclosure more apparent and understandable, the specific implementations of the present disclosure will be explained in detail below in conjunction with the accompanying drawings. In the following description, numerous specific details are set forth in order to facilitate a thorough understanding of the present disclosure. However, the present disclosure can be implemented in many other ways different from those described herein, and those skilled in the art can make similar modifications without departing from the spirit of the disclosure, so that the present disclosure is not limited to the specific embodiments disclosed below.

In the description of the present disclosure, it is to be understood that the terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", etc. indicate the orientations or positional relationships on the basis of the drawings. These terms are only intended for facilitating illustrating the present disclosure and simplifying the illustration, rather than indicating or implying that the devices or elements referred thereto have to present particular orientations, and be constructed and operated in particular orientations, and therefore cannot be construed as limiting the present disclosure.

In addition, the terms "first" and "second" are only intended for illustrative purposes, rather than being construed as indicating or implying relative importance or implicitly designating the number of the technical features as indicated. Thus, the features modified by "first" and "second" may explicitly or implicitly include at least one said feature. In the illustrations of the present disclosure, the term "a plurality of' means at least two, for example two or three, unless otherwise explicitly and specifically defined.

In the present disclosure, unless otherwise expressly specified and defined, the terms "mounted", "connected to", "coupled" and "fixed" should be understood in a broad sense, for example, fixedly connected or detachably connected, or integrated; mechanically connected or electrically connected; directly connected or indirectly connected through an intermediate medium, or in an interior communication or mutual interaction relationship between two elements, unless otherwise specifically defined. For those of ordinary skill in the art, the specific meanings of the above-described terms in the present disclosure may be understood according to specific circumstances.

In the present disclosure, unless otherwise expressly stated and defined, the first feature, when being referred to as being located "above" or "below" the second feature, may be in direct contact with the second feature, or in indirect contact with the second feature via an intermediate feature. Moreover, the first feature, when being referred to as being disposed "on", "above" and "over" the second feature, may be disposed right above or obliquely above the second feature, or simply disposed at a level higher than the second feature. The first feature, when being referred to as being disposed "under", "below" and "beneath" the second feature, may be disposed directly below or obliquely below the second feature, or simply disposed at a level lower than the second feature.

It should be noted that, when an element is referred to as being "fixed to" or "disposed on" another element, it may be directly on the other element or may also be present with an intermediate element. When an element is referred to as being "connected" to another element, it may be directly connected to the other element or might be present with an intermediate element at the same time. The terms "vertical", "horizontal", "up", "down", "left", "right" and similar expressions used herein are only for the purpose of illustration, rather than presenting the only ways for implementation.

Unless otherwise defined, all technical and scientific terms used herein have the same meanings as those commonly understood by those skilled in the art of the present disclosure. The terms used in the description of the present disclosure herein are only for the purpose of describing specific embodiments and are not intended to limit the present disclosure. The term "and/or" used herein includes any and all combinations of one or more related listed items.

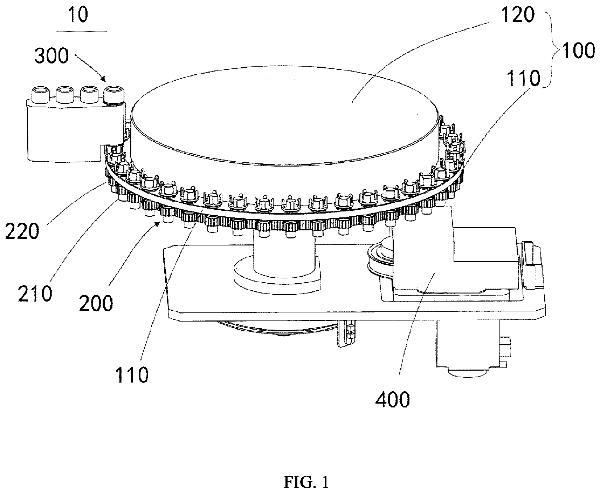

Referring to FIG. 1, an embodiment of the present disclosure provides a reagent mixing and loading device 10.

The reagent mixing and loading device 10 includes a transmission mechanism 100, mixing mechanisms 200, a loading mechanism 300, and a driving mechanism 400.

The transmission mechanism 100 includes a first rotating member 110 and a second rotating member 120.

The first rotating member 110 is rotatably connected to a plurality of spaced apart mixing mechanisms 200, and the plurality of spaced apart mixing mechanisms 200 are distributed along a same circumference. Preferably, the first rotating member 110 is provided with a plurality of receiving holes distributed along the circumference, and the mixing mechanism 200 is provided in each receiving hole.

The first rotating member 110 is sleeved on the second rotating member 120 and can rotate synchronously with the second rotating member 120. The second rotating member 120 may be provided with corresponding functions as required.

The driving mechanism 400 is connected to the second rotating member for driving the second rotating member 120 to rotate.

The mixing mechanism 200 includes a mixing column 210, a mixing transmission member 220, and a mixing limiting member 230. The mixing column 210 is rotatably disposed in the receiving hole. The mixing transmission member 220 is sleeved on and connected to the mixing column 210, and the mixing transmission member 220 abuts against and is engaged with the second rotating member 120, so as to rotate in an opposite direction relative to the second rotating member 120. Since the second rotating part 120 and the mixing transmission member 220 are in abutment fit, such as rolling fit, gear meshing, etc., the mixing transmission member 220 can rotate in the opposite direction with the rotation of the second rotating member 120.

The mixing mechanism 200 may be made of engineering plastics. In this case, the mixing mechanism 200 has a light weight, simple structure, and is easy to mold and shape. It should be understood that, in other embodiments, the preparation material of the mixing mechanism 200 is not limited to the above, and the preparation material of the mixing mechanism 200 may also be a metal material.

The loading mechanism 300 includes a loading box 310 and a loading fitting member 320. The loading box is provided with a reagent hole configured to place the loading box 310. The loading fitting member 320 is rotatably disposed in the reagent hole for carrying the loading box 310. The mixing limiting member 230 can be engaged with the loading fitting member 320 and push the loading fitting member 320 to rotate. Specifically, when loading the loading box 310, the loading box 310 is gripped to the position of the mixing mechanism 200 by a gripping mechanism of the loading box 310, and the mixing limiting member 230 can be engaged with the loading fitting member 320, for example, the two abut against each other. When the mixing limiting member 230 rotates with the mixing column 210, the mixing limiting member 230 can push the mixing limiting member 230 to rotate. The rotation of the mixing limiting member 230 can drive the loading fitting member 320 and the loading box 310 carried by the loading fitting member 320 to rotate, so as to achieve the mixing of reagents.

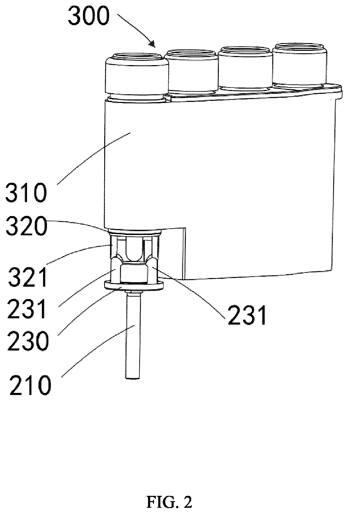

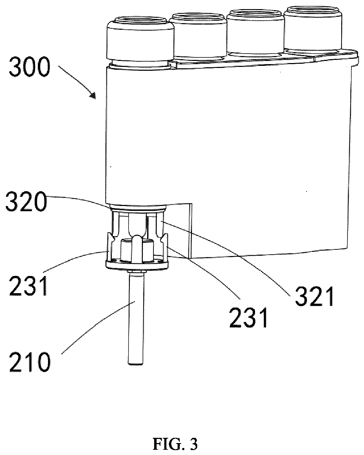

Referring to FIG. 2, in some embodiments, the mixing limiting member 230 includes at least two spaced apart elastic limiting arms 231. The elastic limiting arm 231 is connected to the mixing column 210. The loading fitting member 320 is provided with at least two spaced apart loading limiting arms 321. The loading limiting arm 321 is located at least partially outside the reagent hole. When the loading limiting arm 321 is misaligned with the elastic limiting arm 231, the loading limiting arm 321 abuts against the elastic limiting arm 231. Specifically, during loading, the loading box 310 is gripped to the position of the mixing mechanism 200 by the gripping mechanism of the loading box 310. In this case, the loading limiting arm 321 may not reach a position where the loading limiting arm 321 is completely misaligned with the elastic limiting arm 231, but the loading is not affected. Since the elastic limiting arm 231 has elasticity, when the loading limiting arm 321 moves downward, the loading limiting arm 321 just abuts against the elastic limiting arm 231 (the loading limiting arm 321 is completely aligned with the elastic limiting arm 231, referring to FIG. 2). The loading limiting arm 321 can squeeze and gradually expand the elastic limiting arm 231. With the rotation of the mixing column 210, the loading limiting arm 321 is gradually misaligned with the elastic limiting arm 231 until the loading limiting arm 321 is completely misaligned with the elastic limiting arm 231 (referring to FIG. 3). Meanwhile, the elastic limiting arm 231 resets. The loading limiting arm and the elastic limiting arm 231 abut against each other to generate interference in a radial direction. The loading limiting arm 321 can rotate with the rotation of the elastic limiting arm 231. The rotation of the loading limiting arm 321 can drive the carried loading box 310 to rotate, so as to achieve the mixing of the reagents.

In some embodiments, the loading fitting member 320 also includes a bearing plate. The bearing plate is rotatably provided in the reagent hole, and the loading limiting arm 321 is connected to the bearing plate and protrudes from the reagent hole. For example, the reagent hole is provided along a vertical direction of the loading box 310, and the bearing plate is rotatably provided in the reagent hole, the bearing plate is adjacent to a bottom portion of the reagent hole. An end of the loading limiting arm 321 is connected to a bottom surface of the bearing plate, and another end of the loading limiting arm 321 extends toward an outside of the reagent hole.

In some embodiments, as shown in FIG. 2, a plurality of loading limiting arms 321 are provided. The plurality of loading limiting arms 321 are equally spaced apart on a first circumference.

In some embodiments, as shown in FIG. 2, a plurality of elastic limiting arms 231 are provided. The plurality of elastic limiting arms 231 are equally spaced apart on a second circumference.

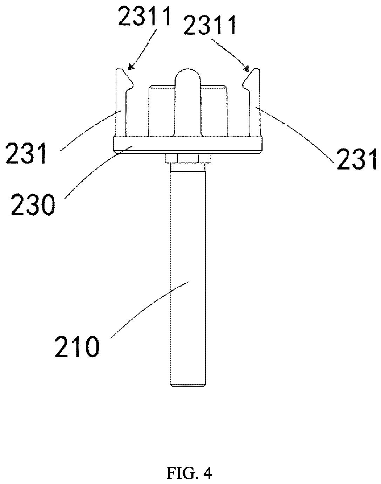

In some embodiments, the loading limiting arm 321 has a certain thickness, and the elastic limiting arm 231 has a certain thickness. Therefore, the first circumference where the loading limiting arm 321 is located is in an annular shape, and the second circumference where the elastic limiting arm 231 is located is also in an annular shape. A maximum diameter of the first circumference is less than a maximum diameter of the second circumference and greater than a minimum diameter of the second circumference. As shown in FIG. 4, an inner wall of an end of the elastic limiting arm 231 facing the loading limiting arm 321 is provided with a guiding yielding surface 2311, and the guiding yielding surface 2311 is in a shape of an outward slope. This arrangement can ensure that the loading limiting arm 321 moves downward directly above the elastic limiting arm 231. When the loading limiting arm 321 and the elastic limiting arm 231 are not completely misaligned (referring to FIG. 2), the loading limiting arm 321 would slowly contact the elastic limiting arm 231. The loading limiting arm 321 moves downward along the guiding yielding surface 2311, and gradually squeeze the elastic limiting arm 231 to be expanded. With the rotation of the mixing column 210, the loading limiting arm 321 is gradually misaligned with the elastic limiting arm 231 until the loading limiting arm 321 is completely misaligned with the elastic limiting arm 231 (referring to FIG. 3).

In some embodiments, the second rotating member 120 is a gear, the mixing transmission member 220 is a mixing gear, and the second rotating member 120 is engaged with the mixing transmission member 220. The second rotating member 120 is engaged with the mixing transmission member 220 to improve transmission stability.

In some embodiments, the transmission mechanism 100 further includes a first transmission wheel, a transmission belt, and a second transmission wheel. The first transmission wheel, the transmission belt and the second transmission wheel are not shown in the drawings. The first transmission wheel is connected to the second transmission wheel through the transmission belt. The second transmission wheel is connected to a driving shaft of the driving mechanism 400. The second transmission wheel is connected to the second rotating member 120 through a transmission shaft.

In some embodiments, a plurality of loading mechanisms 300 are provided. The plurality of loading mechanisms 300 can improve the efficiency of reagent loading and realize loading of multiple reagent kits. For example, during the test, some reagents are used up, and different reagents are required for different test items. In these cases, reagent loading or reagent replacement is required. At this time, no shutdown is required. Different reagent kit can be loaded through the gripping mechanism of reagent kit. Reloading is not prone to problems such as stuck and collision, which does not affect ongoing testing.

In some embodiments, the reagent mixing and loading device 10 further includes a loading transfer mechanism connected to the loading box 310 for driving the loading box 310 to move.

The above reagent mixing and loading device 10 can not only realize the normal mixing of reagents, but also realize the smooth introduction of reagent kit without stuck and collision to speed up the test progress.

An embodiment of the present disclosure further provides an immunological detection apparatus.

The immunological detection apparatus includes the reagent mixing and loading device 10.

In summary, the above reagent mixing and loading device 10 has the following beneficial effects:
(1) The mixing mechanism 200 is simple in structure, and the mixing transmission member 220 of the mixing mechanism 200 has elasticity. In actual production, it can be produced by opening a mold taking the advantage of the elasticity of plastic materials, and a large-scale production can be performed by opening a plastic mold, which is low in price and cost.
(2) Without considering the transmission error and strictly limiting the transmission ratio of the large and small gears (i.e., the second rotating member 120 and the mixing transmission member 220), and regardless of the relative state of the elastic limiting arm 231 and the loading limiting arm 321 of the loading mechanism 300, the loading and introduction of the loading box 310 can be realized without stuck and collision.
(3) In the above reagent mixing and loading device 10, the maximum diameter of the first circumference is less than the maximum diameter of the second circumference and greater than the minimum diameter of the second circumference. In this way, it can be ensured that when the loading limiting arm 321 is loading, an entering position of the mixing transmission member 220 is within the end surface of the elastic limiting arm 231. Since the inner wall of the end of the elastic limiting arm 231 facing the loading limiting arm 321 has the guiding yielding surface 2311, the loading limiting arm 321 can gradually move downward along the slope of the guiding yielding surface 2311 to push away the elastic limiting arm 231. With the rotation of the mixing column 210, the loading limiting arm 321 and the elastic limiting arm 231 can gradually be in a mutually misaligned and clamped state. At this time, the squeezing force exerted by the loading limiting arm 321 on the elastic limiting arm 231 disappears, and the elastic limiting arm 231 is reset. With the rotation of the mixing column 210, the elastic limiting arm 231 pushes the loading limiting arm 321 to rotate, so as to achieve mixing.
(4) During loading, the forces exerted on the elastic limiting arm 231 is gradually reduced by multiple small distance movements of the loading limiting arm 321, and the deformation amount is also gradually reduced, so that the elastic limiting arm 231 is prevented from being directly subjected to a relatively large impact force, the reagent loading is smoother, and the service life of the elastic limiting arm 231 is also prolonged.

The technical features of the embodiments above may be combined arbitrarily. To make the description concise, not all possible combinations of the technical features in the above embodiments are described. However, as long as there are no contradictions in the combinations of these technical features, all of the combinations should be considered to be within the scope of the specification.

The embodiments above only represent several implementation modes of the present disclosure, and the description thereof is relatively specific and detailed, but it should not be construed as limiting the scope of the patent. It should be noted that for those skilled in the art, various modifications and improvements may be made without departing from the concept of the present disclosure, and all these modifications and improvements belong to the protection scope of the present disclosure. Therefore, the scope of protection of the patent disclosure should be subject to the appended claims.

## Claims

1. A reagent mixing and loading device, comprising a transmission mechanism, mixing mechanisms, a loading mechanism and a driving mechanism,
wherein the transmission mechanism comprises a first rotating member and a second rotating member, the first rotating member is rotatably connected to a plurality of spaced apart mixing mechanisms, the first rotating member is sleeved on the second rotating member and is capable of rotating synchronously with the second rotating member, the driving mechanism is connected to the second rotating member to drive the second rotating member to rotate;
the mixing mechanism comprises a mixing column, a mixing transmission member, and a mixing limiting member, the mixing transmission member is sleeved on and connected to the mixing column, and the mixing transmission member abuts against and is engaged with the second rotating member, so as to rotate in an opposite direction relative to the second rotating member; and
the loading mechanism comprises a loading box and a loading fitting member, the loading box is provided with a reagent hole configured to place the loading box, the loading fitting member is rotatably disposed in the reagent hole to carry the loading box, and the mixing limiting member is capable of being engaged with the loading fitting member and pushing the loading fitting member to rotate.

2. The reagent mixing and loading device according to claim 1, wherein the mixing limiting member comprises at least two spaced apart elastic limiting arms , the elastic limiting arm is connected to the mixing column, the loading fitting member is provided with at least two spaced apart loading limiting arms, the loading limiting arm is located at least partially outside the reagent hole, and when the loading limiting arm is misaligned with the elastic limiting arm, the loading limiting arm abuts against the elastic limiting arm.

3. The reagent mixing and loading device according to claim 2, wherein the loading fitting member further comprises a bearing plate, the bearing plate is rotatably provided in the reagent hole, and the loading limiting arm is connected to the bearing plate and protrudes from the reagent hole.

4. The reagent mixing and loading device according to claim 3, wherein a plurality of loading limiting arms are provided, and the plurality of loading limiting arms are equally spaced apart on a first circumference.

5. The reagent mixing and loading device according to claim 4, wherein a plurality of elastic limiting arms are provided, and the plurality of elastic limiting arms are equally spaced apart on a second circumference.

6. The reagent mixing and loading device according to claim 5, wherein a maximum diameter of the first circumference is less than a maximum diameter of the second circumference and greater than a minimum diameter of the second circumference, and an inner wall of an end of the elastic limiting arm facing the loading limiting arm is provided with a guiding yielding surface, and the guiding yielding surface is in a shape of an outward slope.

7. The reagent mixing and loading device according to any one of claims 1 to 6, wherein the second rotating member is a gear, the mixing transmission member is a mixing gear, and the second rotating member is engaged with the mixing transmission member.

8. The reagent mixing and loading device according to any one of claims 1 to 6, wherein the transmission mechanism further comprises a first transmission wheel, a transmission belt, and a second transmission wheel, the first transmission wheel is connected to the second transmission wheel through the transmission belt, the second transmission wheel is connected to a driving shaft of the driving mechanism, and the second transmission wheel is connected to the second rotating member through a transmission shaft.

9. The reagent mixing and loading device according to any one of claims 1 to 6, wherein a plurality of loading mechanisms are provided, and the reagent mixing and loading device further comprises a loading transfer mechanism connected to the loading box to drive the loading box to move.

10. An immunological detection apparatus, comprising the reagent mixing and loading device according to any one of claims 1 to 9.
